# EUROPEAN PATENT APPLICATION

(11) **EP 4 691 503 A1**
(43) Date of publication of application: **11.02.2026**
(21) Application number: 23930835.6
(22) Date of filing: 16.11.2023
(51) Int. Cl.: A61L 27/00, A61B 17/70, A61L 27/04, A61L 27/08, A61L 27/10, A61L 27/14, A61L 27/18, A61L 27/34, A61L 27/44, A61L 27/50

(54) **INTRASPINAL FIXATION INSTRUMENT ROD**

(30) Priority: 29.03.2023 JP 2023054349
(71) Applicant: Globeride, Inc., Higashikurume-shi, Tokyo 203-8511 (JP)
(72) Inventor: OIKAWA Katsuhiro, Higashikurume-shi Tokyo 203-8511 (JP)
(74) Representative: Boult Wade Tennant LLP
(86) International application number: PCT/JP2023/041292
(87) International publication number: WO 2024/202208

(57) **Abstract**

An object is to provide an intraspinal fixture rod that reduces damage at the time of fixing with a screw, has high rigidity and high durability against a deformation load, and significantly reduces a risk of damage to a human body even at the time of breaking. A fixture rod according to one embodiment of the present invention comprises a core member containing a fiber-reinforced resin, and a coating resin layer coating the core member, and the coating resin layer has an elongation of 30% or more and has a thickness in a range of 0.05 mm to 0.4 mm.

## Description

### Technical Field

### Cross Reference

The present application claims priority based on Japanese Patent Application No. 2023-054349 (filed on March 29, 2023), the contents of which are incorporated herein by reference in their entirety.

The present invention relates to an intraspinal fixture rod (referred to as a fixture rod for convenience in the present specification) used for a fixture for fixing a spine.

### Background Art

Conventionally, a fixture rod using metal as a fixture for fixing a spine has been known.

As such a fixture rod, for example, Patent Literature 1 discloses a spinal pedicle rod including an internally reinforced polymer core at least partially encased in a polymer coating.

### Citation List

### Patent Literature

Patent Literature 1: JP 2011-508623 A

### Summary of Invention

### Technical Problem

A fixture rod using metal is generally excellent in terms of fixing force and strength but has a problem that a magnetic field is affected by magnetization of the metal in the magnetic field at the time of imaging by MRI or the like, image disturbance occurs, and diagnosis based on a captured image is difficult. On the other hand, with the rod disclosed in Patent Literature 1, although there is no such problem, it has been found that it is difficult to impart desired rigidity because a fiber density is low, and not only there is a problem in strength and durability, but also there is a problem in safety because spinate fibers are exposed at the time of breaking.

One object of the present invention is to provide a fixture rod that reduces damage at the time of fixing with a screw, has high rigidity and high durability against a deformation load, and significantly reduces a risk of damage to a human body even at the time of breaking. Other objects of the present invention will become apparent upon reference to the entirety of the present specification.

### Solution to Problem

A fixture rod according to one embodiment of the present invention comprises a core member containing a fiber-reinforced resin, and a coating resin layer coating the core member, and the coating resin layer has an elongation of 30% or more and has a thickness in a range of 0.05 mm to 0.4 mm.

In the fixture rod according to one embodiment of the present invention, a fiber volume content (VF) in the vicinity of a surface layer of the core member is in a range of 50% to 70%.

In the fixture rod according to one embodiment of the present invention, a fiber volume content (VF) in the vicinity of a surface layer of the core member is in a range of 55% to 65%.

In the fixture rod according to one embodiment of the present invention, the core member is formed by laminating a plurality of layers.

In the fixture rod according to one embodiment of the present invention, a resin of the core member and a resin of the coating resin layer are the same thermoplastic resin.

In the fixture rod according to one embodiment of the present invention, a resin of the coating resin layer is formed by welding a resin tube.

In the fixture rod according to one embodiment of the present invention, in the plurality of layers of the core member, a plurality of reinforcing fiber layers and a plurality of layers of any one of a resin layer, a reinforcing fiber layer having fibers different from those of the reinforcing fiber layer, and a reinforcing fiber layer having fibers obliquely oriented with respect to a fiber direction of the reinforcing fiber layer are alternately formed as viewed in a cross-section.

In the fixture rod according to one embodiment of the present invention, the reinforcing fiber layer is a fiber-reinforced resin, uses carbon, glass, boron, SiC, or aramid as a fiber, and uses epoxy, phenol, unsaturated polyester, PA, PC, PPSU, POM, PP, PE, ABS, PS, PAEK, or PEEK as a resin.

In the fixture rod according to one embodiment of the present invention, a resin of the resin layer is epoxy, phenol, unsaturated polyester, PA, PC, PPSU, POM, PP, PE, ABS, PS, PAEK, or PEEK.

In the fixture rod according to one embodiment of the present invention, the reinforcing fiber layer having fibers different from those of the reinforcing fiber layer is a fiber-reinforced resin, uses carbon, glass, boron, SiC, or aramid as a fiber, and uses epoxy, phenol, unsaturated polyester, PA, PC, PPSU, POM, PP, PE, ABS, PS, PAEK, or PEEK as a resin.

In the fixture rod according to one embodiment of the present invention, in the reinforcing fiber layer or the reinforcing fiber layer having fibers different from those of the reinforcing fiber layer, fibers are aligned in one direction, formed in a woven fabric form, or randomly oriented. In addition, in the fixture rod according to one embodiment of the present invention, a fiber direction of the reinforcing fiber layer having fibers obliquely oriented with respect to a fiber direction of the reinforcing fiber layer is obliquely oriented in a range of 10° to 90°.

In the fixture rod according to one embodiment of the present invention, when a plurality of layers of any one of a resin layer, a reinforcing fiber layer having fibers different from those of the reinforcing fiber layer, and a reinforcing fiber layer having fibers obliquely oriented with respect to a fiber direction of the reinforcing fiber layer are provided, the layers are the same layers or different layers.

In the fixture rod according to one embodiment of the present invention, the fibers of the reinforcing fiber layer are long fibers.

In the fixture rod according to one embodiment of the present invention, a fiber content of the reinforcing fiber layer is 60 weight% or more.

In the fixture rod according to one embodiment of the present invention, fiber directions of the reinforcing fiber layer are aligned, and a thickness of the layer is in a range of 0.02 mm to 0.3 mm.

In the fixture rod according to one embodiment of the present invention, a marker is embedded in the core member.

### Advantageous Effects of Invention

According to each of the above embodiments of the present invention, it is possible to provide a fixture rod that reduces damage at the time of fixing with a screw, has high rigidity and high durability against a deformation load, and significantly reduces a risk of damage to a human body even at the time of breaking.

### Brief Description of Drawings

Fig. 1 is a view illustrating a spinal fixture 10 comprising a fixture rod according to one embodiment of the present invention.
Fig. 2 is a view schematically illustrating a cross-section of the fixture rod according to one embodiment of the present invention taken along a plane perpendicular to a central axis of the fixture rod.
Fig. 3 is a view schematically illustrating a cross-section of a core member of the fixture rod according to one embodiment of the present invention taken along a plane perpendicular to a central axis of the core member.
Fig. 4 is a view schematically illustrating a cross-section of a core member of the fixture rod according to one embodiment of the present invention taken along a plane perpendicular to a central axis of the core member.
Fig. 5 is a view schematically illustrating a cross-section of a core member of the fixture rod according to one embodiment of the present invention taken along a plane perpendicular to a central axis of the core member.

### Description of Embodiments

Hereinafter, embodiments of a fixture rod according to the present invention will be specifically described with reference to the accompanying drawings. Constituent elements common in the plurality of drawings are assigned with the same reference signs throughout the plurality of drawings. It should be noted that each of the drawings is not necessarily drawn to an accurate scale for convenience of description.

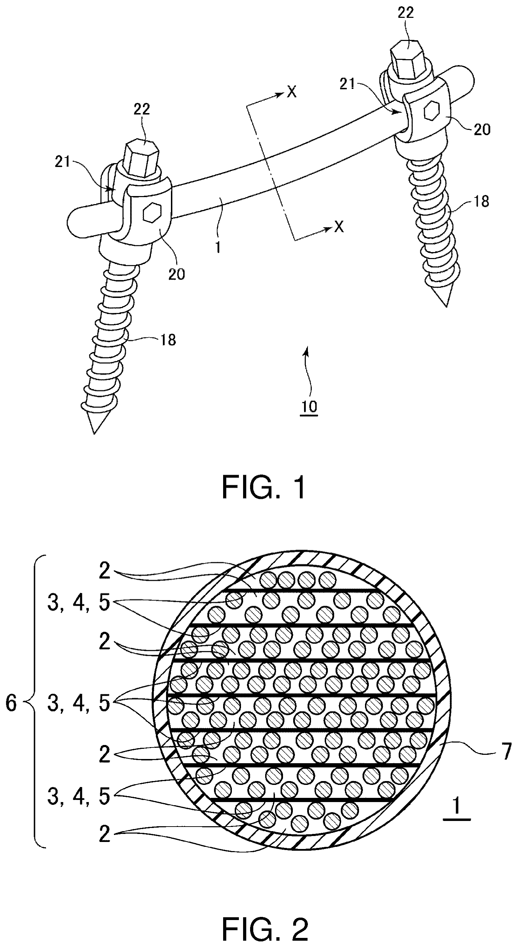

Fig. 1 is a view illustrating a spinal fixture 10 comprising a fixture rod 1 according to one embodiment of the present invention. As illustrated in the drawing, the spinal fixture 10 comprises: a plurality of screw members 18 (two screw members 18 in the illustrated example) to be fixed to a bone of a spine; a plurality of rod fixing members 20 (two rod fixing members 20 in the illustrated example) attached to the screw members 18, each of the rod fixing members 20 comprising a recess 21 for receiving the fixture rod 1 and a pressing member 22; and the fixture rod 1 inserted into the recesses 21 of the plurality of rod fixing members 20 and fixed by the pressing members 22.

Next, the fixture rod 1 according to one embodiment of the present invention used for the spinal fixture 10 will be described with reference to Fig. 2. Fig. 2 is a cross-sectional view of the fixture rod 1 illustrated in Fig. 1 taken along line X-X illustrated in Fig. 1. The fixture rod 1 according to one embodiment of the present invention comprises a core member 6 containing a fiber-reinforced resin, which will be described in detail later, and a coating resin layer 7 coating the core member 6, and the coating resin layer 7 has an elongation of 30% or more and has a thickness in a range of 0.05 mm to 0.4 mm. By inclusion of such a coating resin layer 7, even when the core member 6 inside is damaged, it is possible to ensure a state in which the core member 6 is coated without damaging the coating resin layer 7. As a result, it is possible to prevent exposure of fibers of the core member 6 to the outside, and to significantly reduce a risk of damage to a human body even at the time of breakage. More particularly, the elongation of the coating resin is sufficiently large relative to a standard elongation of about 2% of carbon fibers, and therefore breakage occurs from the carbon fibers. In addition, when the coating resin has a certain thickness and strength or more, breakage of the coating resin can be suppressed against an impact force due to breakage of the carbon fibers. However, when the thickness of the coating resin is in a range exceeding 0.4 mm, breakage of the coating hardly occurs, but the outer diameter itself of the rod increases, and an influence on indwelling in a body increases. In addition, the core member described later can reduce damage at the time of fixing with a screw and can improve durability against a deformation load because of high rigidity, but the coating resin layer further exhibits the above technical effects. Here, in the fixture rod 1 according to one embodiment of the present invention, a marker is embedded in the core member 6. Note that the marker is made of a radiopaque substance and plays a role of indicating the position of an implant product in a body.

In the fixture rod 1 according to one embodiment of the present invention, a fiber volume content (VF) in the vicinity of a surface layer of the core member 6 is preferably in a range of 50% to 70%, and more preferably in a range of 55% to 65%. Here, the vicinity of the surface layer of the core member 6 refers to a region 0.2 mm inward from a surface of the core member in a center direction. When the fiber volume content (VF) in the vicinity of the surface layer of the core member 6 is 50% or more, bending rigidity can be efficiently increased, and a rod that is hardly damaged can be formed. When the volume ratio in the vicinity of the surface of the core member is 50% or more, bonding to the coating resin is not completely integrated. That is, while a resin on the surface of the core member and the coating resin are easily firmly integrated, carbon fibers on the surface of the core member and the coating resin are relatively weakly bonded to each other. On the other hand, when the fiber volume content (VF) in the vicinity of the surface layer of the core member 6 exceeds 70%, the amount of resin on the surface is too small, and therefore sufficient bonding cannot be obtained between the coating resin and the resin of the core member. Since the core member and the coating resin are not completely integrated but are weakly bonded to each other to a certain extent or more, when the core member inside is damaged, a crack develops between the surface of the core member and the coating resin layer to release stress, and therefore breakage of the coating resin hardly occurs.

In the fixture rod 1 according to one embodiment of the present invention, the resin of the core member 6 and the resin of the coating resin layer 7 are the same thermoplastic resin. Examples of such a thermoplastic resin include PEEK, nylon, PPSU, and PET. In this way, the core member and the coating resin can be stably integrated.

In the fixture rod 1 according to one embodiment of the present invention, a resin of the coating resin layer is formed by welding a resin tube. By molding the coating layer into a tubular shape in advance in this way, the coating layer having a stable thickness and quality can be formed.

In the fixture rod 1 according to one embodiment of the present invention, the core member 6 is formed by laminating a plurality of layers, and details thereof will be described later.

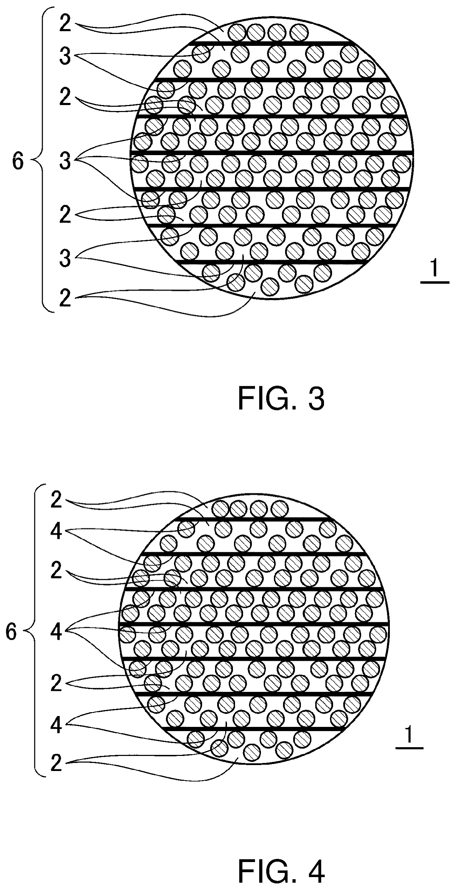

Next, the plurality of layers of the core member 6 of the fixture rod 1 according to one embodiment of the present invention used for the spinal fixture 10 will be described with reference to Figs. 3, 4, and 5. Each of Figs. 3, 4, and 5 is a cross-sectional view of the core member 6 of the fixture rod 1 illustrated in Fig. 1 taken along line X-X illustrated in Fig. 1. Note that, for convenience of description, the above-described coating resin layer 7 is omitted.

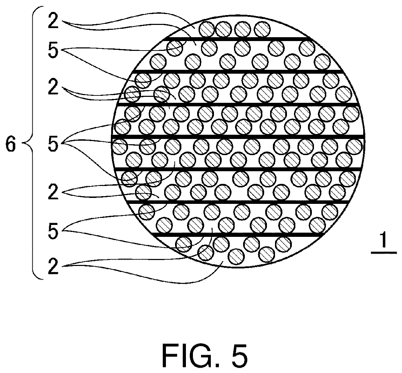

As illustrated in the drawings, in the plurality of layers of the core member 6 of the fixture rod 1 according to one embodiment of the present invention, a plurality of reinforcing fiber layers 2 (each of eight reinforcing fiber layers in the example of Fig. 3 or 5) and a plurality of layers of any one of a resin layer 3 (each of seven resin layer 3 in the example illustrated in Fig. 3), a reinforcing fiber layer 4 (each of seven reinforcing fiber layers 4 in the example illustrated in Fig. 4) having fibers different from those of the reinforcing fiber layer 2, and a reinforcing fiber layer 5 (each of seven reinforcing fiber layers 5 in the example illustrated in Fig. 5) having fibers obliquely oriented with respect to a fiber direction of the reinforcing fiber layer 2 are alternately formed as viewed in a cross-section. Here, each of the reinforcing fiber layer 2 and any one of the resin layer 3 (example illustrated in Fig. 3), the reinforcing fiber layer 4 having fibers different from those of the reinforcing fiber layer (example illustrated in Fig. 4), and the reinforcing fiber layer 5 having fibers obliquely oriented with respect to the fiber direction of the reinforcing fiber layer (example illustrated in Fig. 5) is formed to have a thickness of 0.01 mm to 0.25 mm. However, the thickness of each of the layers may vary depending on a location in a layer (that is, the layers may be formed such that there may be a portion having a small layer thickness and a portion having a large layer thickness). In addition, the layers may be formed intermittently (that is, not only there may be a portion having a small layer thickness and a portion having a large layer thickness, but also a portion having a layer thickness of zero may be interposed). Here, the reinforcing fiber layer 5 is a fiber-reinforced resin, can use carbon, glass, boron, SiC, or aramid as fibers, and can use epoxy, phenol, unsaturated polyester, PA, PC, PPSU, POM, PP, PE, ABS, PS, PAEK, or PEEK as a resin.

The core member 6 of the fixture rod 1 according to one embodiment of the present invention can provide a fixture rod that reduces damage at the time of fixing with a screw, has high rigidity and high durability against a deformation load, and significantly improves safety even at the time of breaking. Particularly, it has been found that at the time of breaking of the fixture rod 1 according to one embodiment of the present invention, by concentrating stress on any one of the resin layer, the reinforcing fiber layer having fibers different from those of the reinforcing fiber layer, and the reinforcing fiber layer having fibers obliquely oriented with respect to the fiber direction of the reinforcing fiber layer, which are sandwiched between the reinforcing fiber layers, a chance of the reinforcing fiber layer becoming spinate when the rod is broken is reduced, and as a result, it is possible to significantly enhance safety to a human body.

In the core member 6 of the fixture rod 1 according to one embodiment of the present invention, the reinforcing fiber layer 2 is a fiber-reinforced resin, uses carbon, glass, boron, SiC, or aramid as fibers, and uses epoxy, phenol, unsaturated polyester, PA, PC, PPSU, POM, PP, PE, ABS, PS, PAEK, or PEEK as a resin. In this way, it is possible to increase bending rigidity and strength of the fixture rod 1.

In the core member 6 of the fixture rod 1 according to one embodiment of the present invention, the resin of the resin layer 3 is epoxy, phenol, unsaturated polyester, PA, PC, PPSU, POM, PP, PE, ABS, PS, PAEK, or PEEK.

In the core member 6 of the fixture rod 1 according to one embodiment of the present invention, the reinforcing fiber layer 4 having fibers different from those of the reinforcing fiber layer 2 is a fiber-reinforced resin, uses carbon, glass, boron, SiC, or aramid as fibers, and uses epoxy, phenol, unsaturated polyester, PA, PC, PPSU, POM, PP, PE, ABS, PS, PAEK, or PEEK as a resin. In this way, it is possible to increase the bending rigidity and the strength of the fixture rod. A reason for using a reinforcing fiber layer having fibers different from those of the reinforcing fiber layer 2 is that different characteristics such as flexibility and vibration absorbing properties other than rigidity and strength can be imparted by using different materials.

In the core member 6 of the fixture rod 1 according to one embodiment of the present invention, in the reinforcing fiber layer 2 or the reinforcing fiber layer 4 having fibers different from those of the reinforcing fiber layer 2, the fibers may be aligned in one direction, formed in a woven fabric form, or randomly oriented. In addition, in the core member 6 the fixture rod 1 according to one embodiment of the present invention, the fiber direction of the reinforcing fiber layer 5 having fibers obliquely oriented with respect to the fiber direction of the reinforcing fiber layer 2 may be obliquely oriented in a range of 10° to 90°. In this way, it is possible to achieve intended strength and rigidity by disposing an appropriate laminated configuration.

In the core member 6 of the fixture rod 1 according to one embodiment of the present invention, when a plurality of layers of any one of the resin layer 3, the reinforcing fiber layer 4 having fibers different from those of the reinforcing fiber layer 2, and the reinforcing fiber layer 5 having fibers obliquely oriented with respect to the fiber direction of the reinforcing fiber layer 2 are provided, the layers are the same layers or different layers (hereinafter, the same applies). In this regard, for example, when two layers are provided, two layers of the resin layer 3 may be provided (in this case, the two layers are the same), or two different layers such as the resin layer 3 and the reinforcing fiber layer 4 having fibers different from those of the reinforcing fiber layer may be provided. In a case of providing three or more layers, a desired combination may be selected from among the above layers and provided. In this way, it is possible to achieve intended strength and rigidity by disposing an appropriate laminated configuration.

In the core member 6 of the fixture rod 1 according to one embodiment of the present invention, the fibers of the reinforcing fiber layer 2 are long fibers. In this way, since the fibers of the reinforcing fiber layer 2 are long fibers, it is possible to further increase bending rigidity and strength.

In addition, in the core member 6 of the fixture rod 1 according to one embodiment of the present invention, a fiber content of the reinforcing fiber layer 2 is 50% by volume or more. In this way, it is possible to form the fixture rod 1 having high rigidity and excellent durability with the fiber layer in which the long fibers are focused at a high density.

In the core member 6 of the fixture rod 1 according to one embodiment of the present invention, the fiber directions of the reinforcing fiber layer 2 are aligned, and a thickness of the reinforcing fiber layer 2 is, for example, in a range of 0.02 mm to 0.3 mm. In this way, a density of the resin is made uniform, and it is possible to reduce a variation in strength depending on a site.

Next, a method for manufacturing the fixture rod 1 according to one embodiment of the present invention will be described. First, as step 1, any one of the reinforcing fiber layer 2, the resin layer 3, the reinforcing fiber layer 4 having fibers different from those of the reinforcing fiber layer, and the reinforcing fiber layer 5 having fibers obliquely oriented with respect to the fiber direction of the reinforcing fiber layer is cut to predetermined dimensions (layer cutting step). Then, as step 2, the layers are laminated in a predetermined arrangement (layer laminating step). As step 3, the laminated product is set in a predetermined mold and molded under predetermined conditions (for example, temperature: 380°C, pressure: 5 to 15 MPa) (molding step). Next, as step 4, the molded product is processed into a final shape and final dimensions to form the core member 6 (processing step). Finally, as step 5, a resin tube is welded to form the coating resin layer 7 on an outer surface of the formed core member 6. In this way, the fixture rod 1 according to one embodiment of the present invention is formed. Note that, in place of the above method for manufacturing the fixture rod 1, it is possible to form the fixture rod 1 according to one embodiment of the present invention even by a method (pultrusion molding method) in which a plurality of sets of reinforcing fiber bundles impregnated with a liquid resin is prepared, and these bundles are drawn into a mold and caused to pass through the mold to be heated and cured or cooled and solidified. Either a thermosetting resin or a thermoplastic resin may be used.

The fixture rod 1 according to one embodiment of the present invention formed in this manner can provide a fixture rod that reduces damage at the time of fixing with a screw, has high rigidity and high durability against a deformation load, and significantly reduces a risk of damage to a human body even at the time of breaking. More specifically, when the core member is damaged, a crack thereof develops between a surface of the core member and the coating resin layer, and stress is released, whereby breakage of the coating resin hardly occurs. In this way, scattering of fragments and fibers due to breakage of a main body is suppressed.

Dimensions, materials, and arrangements of the components described in the present specification are not limited to those explicitly described in the embodiments, and the components can be modified so as to have any dimensions, materials, and arrangements that can fall within the scope of the present invention. In addition, components not explicitly described in the present specification can also be added to the described embodiments, or some of the components described in the embodiments can be omitted.

### Reference Signs List

1 Fixture rod (intraspinal fixture rod)
2 Reinforcing fiber layer
3 Resin layer
4 Reinforcing fiber layer having fibers different from those of reinforcing fiber layer 2
5 Reinforcing fiber layer having fibers obliquely oriented with respect to fiber direction of reinforcing fiber layer 2
6 Core member
7 Coating resin layer
10 Spinal fixture
18 Screw member
20 Rod fixing member
21 Recess
22 Pressing member

## Claims

1. A fixture rod comprising:
a core member containing a fiber-reinforced resin; and
a coating resin layer coating the core member, wherein
the coating resin layer has an elongation of 30% or more and has a thickness in a range of 0.05 mm to 0.4 mm.

2. The fixture rod according to claim 1, wherein a fiber volume content (VF) in a vicinity of a surface layer of the core member is in a range of 50% to 70%.

3. The fixture rod according to claim 1, wherein a fiber volume content (VF) in a vicinity of a surface layer of the core member is in a range of 55% to 65%.

4. The fixture rod according to claim 1, wherein the core member is formed by laminating a plurality of layers.

5. The fixture rod according to claim 1, wherein a resin of the core member and a resin of the coating resin layer are the same thermoplastic resin.

6. The fixture rod according to claim 1, wherein a resin of the coating resin layer is formed by welding a resin tube.

7. The fixture rod according to claim 4, wherein in the plurality of layers of the core member, a plurality of reinforcing fiber layers and a plurality of layers of any one of a resin layer, a reinforcing fiber layer having fibers different from those of the reinforcing fiber layer, and a reinforcing fiber layer having fibers obliquely oriented with respect to a fiber direction of the reinforcing fiber layer are alternately formed as viewed in a cross-section.

8. The fixture rod according to claim 7, wherein the reinforcing fiber layer is a fiber-reinforced resin, uses carbon, glass, boron, SiC, or aramid as fibers, and uses epoxy, phenol, unsaturated polyester, PA, PC, PPSU, POM, PP, PE, ABS, PS, PAEK, or PEEK as a resin.

9. The fixture rod according to claim 7, wherein a resin of the resin layer is epoxy, phenol, unsaturated polyester, PA, PC, PPSU, POM, PP, PE, ABS, PS, PAEK, or PEEK.

10. The fixture rod according to claim 7, wherein the reinforcing fiber layer having fibers different from those of the reinforcing fiber layer is a fiber-reinforced resin, uses carbon, glass, boron, SiC, or aramid as fibers, and uses epoxy, phenol, unsaturated polyester, PA, PC, PPSU, POM, PP, PE, ABS, PS, PAEK, or PEEK as a resin.

11. The fixture rod according to claim 7, wherein in the reinforcing fiber layer or the reinforcing fiber layer having fibers different from those of the reinforcing fiber layer, fibers are aligned in one direction, formed in a woven fabric form, or randomly oriented.

12. The fixture rod according to claim 7, wherein a fiber direction of the reinforcing fiber layer having fibers obliquely oriented with respect to the fiber direction of the reinforcing fiber layer is obliquely oriented in a range of 10° to 90°.

13. The fixture rod according to claim 7, wherein when a plurality of layers of any one of a resin layer, a reinforcing fiber layer having fibers different from those of the reinforcing fiber layer, and a reinforcing fiber layer having fibers obliquely oriented with respect to the fiber direction of the reinforcing fiber layer are provided, the layers are the same layers or different layers.

14. The fixture rod according to claim 7, wherein fibers of the reinforcing fiber layer are long fibers.

15. The fixture rod according to claim 7, wherein a fiber content of the reinforcing fiber layer is 50% by volume or more.

16. The fixture rod according to claim 7, wherein fiber directions of the reinforcing fiber layer are aligned, and a thickness of the reinforcing fiber layer is in a range of 0.02 mm to 0.3 mm.

17. The fixture rod according to claim 1, wherein a marker is embedded in the core member.
